# EUROPEAN PATENT SPECIFICATION

(11) **EP 2 912 173 B1**
(45) Date of publication and mention of the grant of the patent: **13.11.2019**
(21) Application number: 13760127.4
(22) Date of filing: 10.07.2013
(51) Int. Cl.: C12N 15/03, C12N 15/70

(54) **PROCESS FOR PREPARATION AND PURIFICATION OF RECOMBINANT HUMAN ONCOSTATIN M PROTEIN**
VERFAHREN ZUR HERSTELLUNG UND REINIGUNG VOM REKOMBINANTEN HUMANEN ONCOSTATIN M
PROCÉDÉ DE PRÉPARATION ET PURIFICATION DE LA ONCOSTATIN M HUMAINE RECOMBINANTE

(30) Priority: 23.10.2012 LT 2012099
(43) Date of publication of application: 02.09.2015
(73) Proprietor: UAB Biotechnologines Farmacijos Centras "Biotechpharma", 08412 Vilnius (LT)
(72) Inventor: BUMELIS, Vladas Algirdas, 10103 Vilnius (LT); SUDZIUVIENE, Saule, 09118 Vilnius (LT); MAKAUSKAS, Nerijus, 19100 Anciunu km., Sirvintu raj. (LT); PUKAITE, Lina, 07119 Vilnius (LT)
(74) Representative: Gerasimovic, Liudmila
(86) International application number: PCT/LT2013/000012
(87) International publication number: WO 2014/065642

(56) References cited:
- US-A- 5 047 504
- US-A- 5 047 504
- US-A- 5 428 012
- SPORENO E ET AL: "Production and structural characterization of amino terminally histidine tagged human oncostatin M in E. Coli", CYTOKINE, ACADEMIC PRESS LTD, PHILADELPHIA, PA, US, vol. 6, no. 3, 1 May 1994 (1994-05-01), pages 255-264, XP023271484, ISSN: 1043-4666, DOI: 10.1016/1043-4666(94)90021-3 [retrieved on 1994-05-01]
- Fré Dé ET AL: "The Proteomics of N-terminal Methionine Cleavage* - S", , 1 January 2006 (2006-01-01), XP055308007, Retrieved from the Internet: URL:http://citeseerx.ist.psu.edu/viewdoc/d ownload?doi=10.1.1.320.1011&rep=rep1&type= pdf [retrieved on 2016-10-05]
- KONG N ET AL: "Pilot-scale fermentation, purification, and characterization of recombinant human Oncostatin M in Pichia pastoris", PROTEIN EXPRESSION AND PURIFICATION, ACADEMIC PRESS, SAN DIEGO, CA, vol. 63, no. 2, 1 February 2009 (2009-02-01), pages 134-139, XP025686809, ISSN: 1046-5928, DOI: 10.1016/J.PEP.2008.10.002 [retrieved on 2008-10-14]
- SPORENO E ET AL: "Production and structural characterization of amino terminally histidine tagged human oncostatin M in E. Coli", CYTOKINE, ACADEMIC PRESS LTD, PHILADELPHIA, PA, US, vol. 6, no. 3, 1 May 1994 (1994-05-01), pages 255-264, XP023271484, ISSN: 1043-4666, DOI: 10.1016/1043-4666(94)90021-3 [retrieved on 1994-05-01]
- BRUCE A G ET AL: "Oncostatin M", PROGRESS IN GROWTH FACTOR RESEARCH, PERGAMON PRESS, GB, vol. 4, no. 2, 1 January 1992 (1992-01-01) , pages 157-170, XP023261035, ISSN: 0955-2235, DOI: 10.1016/0955-2235(92)90029-H [retrieved on 1992-01-01]
- MALIK N ET AL: "MOLECULAR CLONING, SEQUENCE ANALYSIS, AND FUNCTIONAL EXPRESSION OF A NOVEL GROWTH REGULATOR, ONCOSTATION M", MOLECULAR AND CELLULAR BIOLOGY, AMERICAN SOCIETY FOR MICROBIOLOGY, WASHINGTON, US, vol. 9, no. 7, 1 July 1989 (1989-07-01), pages 2847-2853, XP000037383, ISSN: 0270-7306
- SPORENO E ET AL: "Production and structural characterization of amino terminally histidine tagged human oncostatin M in E. Coli", CYTOKINE, ACADEMIC PRESS LTD, PHILADELPHIA, PA, US, vol. 6, no. 3, 1 May 1994 (1994-05-01), pages 255-264, XP023271484, ISSN: 1043-4666, DOI: 10.1016/1043-4666(94)90021-3 [retrieved on 1994-05-01]
- Fré Dé ET AL: "The Proteomics of N-terminal Methionine Cleavage* - S", , 1 January 2006 (2006-01-01), XP055308007, Retrieved from the Internet: URL:http://citeseerx.ist.psu.edu/viewdoc/d ownload?doi=10.1.1.320.1011&rep=rep1&type= pdf [retrieved on 2016-10-05]
- KONG N ET AL: "Pilot-scale fermentation, purification, and characterization of recombinant human Oncostatin M in Pichia pastoris", PROTEIN EXPRESSION AND PURIFICATION, ACADEMIC PRESS, SAN DIEGO, CA, vol. 63, no. 2, 1 February 2009 (2009-02-01), pages 134-139, XP025686809, ISSN: 1046-5928, DOI: 10.1016/J.PEP.2008.10.002 [retrieved on 2008-10-14]

## Description

### FIELD OF THE INVENTION

The present invention relates to an immunomodulatory protein useful as antiviral and antitumor agent. The present invention also relates to inserting a gene, encoding native mature protein, in a suitable host; producing the culture of recombinant strain and stimulating expression of the heterologous polypeptide and its accumulation into insoluble inclusion bodies. Particularly, the present invention discloses a process for preparation of human mature Oncostatin M protein using a corresponding gene, encoding native mature OSM protein, inserted in a recombinant Escherichia coli strain, thus providing a N-methionylated, mature form of protein. The invention also relates to a process for isolating and purifying said protein from insoluble fraction of bacterial strain during solubilizing, oxidizing and subjecting the material to chromatography. The preparation of recovered protein is formulated for stability.

### BACKGROUND OF THE INVENTION

Oncostatin M (OSM) was initially identified as a polypeptide cytokine which inhibited the in vitro growth of cells from melanoma and other solid tumors. OSM shows significant similarities in primary amino acid sequence and predicted secondary structure to leukemia inhibitory factor (LIF), ciliary neurotrophic factor (CNTF), granulocyte colony-stimulating factor (G-CSF), interleukin 6 (IL-6), and interleukin 11 (IL-11). Analysis of the genes encoding these proteins reveals a shared exon organization suggesting evolutionary descent from a common ancestral gene. Recent data indicates that OSM also shares a number of in vitro activities with other members of this cytokine family. The overlapping biological effects appear to be explained by the sharing of receptor subunits. [Bruce AG, Linsley PS, Rose TM. Oncostatin M. Prog Growth Factor Res. 1992;4(2):157-70].

The human form of OSM was originally isolated in 1986 from the growth media of PMA treated U-937 histiocytic lymphoma cells by its ability to inhibit the growth of cell lines established from melanomas and other solid tumours [Zarling JM, Shoyab M, Marquardt H, Hanson MB, Lioubin MN, Todaro GJ (December 1986). "Oncostatin M: a growth regulator produced by differentiated histiocytic lymphoma cells". Proc. Natl. Acad. Sci. U.S.A. 83 (24): 9739-43]. A robust protein, OSM is stable between pH 2 and 11 and resistant to heating for one hour at 56 °C. A partial amino acid sequence allowed the isolation of human OSM cDNA and subsequently genomic clones [Malik N, Kallestad JC, Gunderson NL, Austin SD, Neubauer MG, Ochs V, Marquardt H, Zarling JM, Shoyab M, Wei CM (July 1989). "Molecular cloning, sequence analysis, and functional expression of a novel growth regulator, oncostatin M". Mol. Cell. Biol. 9 (7): 2847-53]. The full cDNA clone of hOSM encodes a 252 amino acid precursor, the first 25 amino acids of which functions as a secretory signal peptide, which on removal yields the soluble 227 amino acid pro-OSM. Cleavage of the C-terminal most 31 residues at a trypsin like cleavage site yields the fully active 196 residue form. Two potential N-glycosylation sites are present in hOSM both of which are retained in the mature form [Linsley PS, Kallestad J, Ochs V, Neubauer M (May 1990). "Cleavage of a hydrophilic C-terminal domain increases growth-inhibitory activity of oncostatin M". Mol. Cell. Biol. 10 (5): 1882-90.; Malik N, Graves D, Shoyab M, Purchio AF (1992). "Amplification and expression of heterologous oncostatin M in Chinese hamster ovary cells". DNA Cell Biol. 11 (6): 453-9].

The 196 residue OSM is the predominant form isolated form a variety of cell lines and corresponds to a glycoprotein of 28 KDa, although the larger 227 residue pro-OSM can be isolated from over transfected cells. Pro-OSM although an order of magnitude less efficacious in growth inhibition assays, displays similar binding affinity toward cells in radio ligand binding assays [Malik N, Graves D, Shoyab M, Purchio AF (1992). "Amplification and expression of heterologous oncostatin M in Chinese hamster ovary cells". DNA Cell Biol. 11 (6): 453-9]. Thus post translational processing may play a significant role in the *in vivo* function of OSM. Like many cytokines OSM is produced from cells by de novo synthesis followed by release through the classical secretion pathway. However, OSM can be released from preformed stores within polymorphonuclear leukocytes on degranulation. [Grenier A, Dehoux M, Boutten A, Arce-Vicioso M, Durand G, Gougerot-Pocidalo MA, Chollet-Martin S (February 1999). "Oncostatin M production and regulation by human polymorphonuclear neutrophils". Blood 93 (4): 1413-21]. It still remains unclear how OSM is targeted to these intracellular compartments.

Among the others, the most available host cells for production of recombinant human OSM is Escherichia coli, from which recombinant oncostatin (OSM) was isolated, purified, characterized and the use of rmetHuOSM in malignancies commonly associated with neutropenic infections during therapy, such as breast cancer, lymphoma, and leukemia was established [Bruce AG, Linsley PS, Rose TM. Oncostatin M. Prog Growth Factor Res. 1992;4(2):157-70].

A number of processes for synthesis, isolation and purification of recombinant OSM in E. coli have been developed.
For example, Sporeno E. et al. ["Production and structural characterization of amino terminally histidine tagged human oncostatin M in E.coli", CYTOKINE, Academic Press Ltd, Philadelphia, PA, US, Vol. 6, No.3, publ. 01/05/1994, pages 255-264] discloses production and purification of OSM modified form, where for the expression of the recombinant target protein the pT7-7 plasmid was introduced in E.coli BL21 cells. The product was tagged OSM protein, carrying unnatural sequence of six N-terminal histidines (6xHis-OSM).
For large scale production and clinical use, there is a need for efficient protein purification procedures to remove host DNA and impurities such that a protein for human administration can meet the necessary requirements whilst retaining its biological activity. Accordingly, chromatographic processes and combinations thereof for the purification of OSM have been developed.
Bacterial producent strain which is versatile in different medium and cultivation temperatures is much requested. The strain mutations responding to disulphide bridge formation and correct protein folding are regulated by adding secondary antibiotics and tuning the cultivation temperature. Thus, the created strain allows obtaining both insoluble and soluble protein form. The purification stage is rapid, comprising of single step chromatography process.

As used herein the term "rmetHuOSM" refers to a protein that is produced by a microorganism that has been transformed with a hOSM gene or modification thereof that encodes a protein having a) an amino acid sequence that is at least substantially identical to the amino acid sequence of native human rmetHuOSM and b) physicochemical properties that are identical to native human OSM.
As used herein the term "rmetHuOSM producing microorganism" refers to a microorganism that has been genetically engineered to produce a protein that possesses biological activity associated with human OSM.
A rmetHuOSM producing microorganism can be generated by introducing a nucleic acid molecule encoding OSM. Suitably the OSM-encoding sequence may be part of an expression vector. Preferably, the OSM sequence is the codon modified OSM sequence described herein.
The choice of vector e.g. a plasmid, cosmid, or phage vector will often depend on the host cell into which it is to be introduced. Suitable vectors include pET21a+, pUC57/T and pT7a as described herein.

The vectors for use in the present invention may contain one or more selectable marker genes- such as a gene, which confers antibiotic resistance e.g. ampicillin, kanamycin, chloramphenicol or tetracyclin resistance.
The vector may further comprise a nucleotide sequence enabling the vector to replicate in the host cell in question. Examples of such sequences are the origins of replication of plasmids pUC19, pACYC177, pUBHO, pE194, pAMBI, pIJ702 and pETII.

In some applications, the nucleotide sequence for use in the present invention is operably linked to a regulatory sequence which is capable of providing for the expression of the nucleotide sequence, such as by the chosen host cell.
Preferably, the nucleotide sequence according to the present invention is operably linked to at least a promoter.
Preferably, the host cells are prokaryotic cells. Examples of suitable bacterial host organisms are gram positive or gram negative bacterial species.

The genotype of the host cell may be modified to improve expression.
For example, the host cell E. coli may over-express rare tRNA's to improve expression of heterologous proteins as exemplified/described in Kane [Curr Opin Biotechnol (1995), 6, 494-500 "Effects of rare codon clusters on high-level expression of heterologous proteins in E. coli"]. The host cell may be deficient in a number of reducing enzymes thus favouring formation of stable disulphide bonds as exemplified/described in Bessette [Proc Natl Acad Sci USA (1999), 96, 13703-13708 " Efficient folding of proteins with multiple disulphide bonds in the Escherichia coli cytoplasm"].
Suitable bacterial host cells include E. coli. Suitably E. coli strains include Rosetta gami-2, JMI09 and K802, as described herein.

By "isolated" and/or "purified" is meant that the OSM protein is in a relatively pure state - e.g. at least about 90% pure, or at least about 95% pure or at least about 98% pure.
For use in the process of the present invention, various separation media can be used.
Various gel filtration supports can be used and are selected from the group comprising: Sephadex G-25, Sephadex G-50, Sephadex G-75, Sephacryl S-200HR, Sephacryl S-100HR, Superose 12, Superose 6, Superdex 75, TSKgel G-2500PW, TSK gel G-3000 PW, Bio-Gel P-60, Bio-Gel P-100 etc. Preferably, Sephadex G-25 is used.

Various cationic exchange chromatography supports can be used and may be selected from the group comprising: SP Sepharose FF, SP Sepharose HP, CM Sepharose FF, TSKgel SP-5PW, TSK gel SP-5PW-HR, Toyopearl SP-650M, Toyopearl SP-650S,Toyopearl SP-650C, Toyopearl CM-650M,Toyopearl CM-650S, Macro-Prep High S support, Macro-Prep S support, Macro-Prep CM support etc.

### DISCLOSURE OF THE INVENTION

### Summary of the Invention

The principal object of the present invention is a process for preparation and purification of recombinant human oncostatin M protein (OSMnat), which comprises the following steps:
i) cultivating recombinant Escherichia coli containing a OSMnat gene,
ii) culturing said recombinant Escherichia coli in complex culture medium to produce OSMnat protein, and
iii) isolating and purifying recombinant OSMnat protein from inclusion bodies or soluble protein fraction.
Said OSM gene comprises the sequence SEQ ID NO:1.

According to the process of the present invention recombinant Escherichia coli containing a OSMnat gene is cultivated by amplifying said gene and cloning said amplified modified OSMnat gene into an intermediate or expression vector, amplifying and isolating said OSMnat gene from said vector cloned and transforming said expression vector into said Escherichia coli. Escherichia coli is selected from the group, consisting of Escherichia coli JM109, Escherichia coli BL21 (DE3), Escherichia coli K802 and Escherichia coli Rosetta gami-2. Said Escherichia coli has resistance to antibiotics respectably accordingly host strain, such as ampicillin, kanamycin, chloramphenicol and tetracycline.
The suitable vector is selected from the group, consisting of pET21a+, pT7a, pJET1.2blunt, pQE-T7.
According to the present invention said OSMnat gene is cloned in pET21a+ vector downstream to early bacteriophage T7 promoter. The gene is said to be in a reading frame from promoter, lac operon should be upstream from T7 promoter, and the expression of desired construct, containing a OSMnat gene (expression cassete), is stringently controlled and terminated by T7 terminator.
The culture medium is selected from complex media such as LB, YPD, TB, M9 or M9m, wherein said culture medium comprises one or more nitrogen sources, selected from the group consisting of ammonium salts, nitrates, tryptone, yeast extract and ammonium hydroxide, and a carbon source, selected from the group consisting of glycerol, glucose, fructose and the like, preferably glucose.
The biomass build up is in a range of 2 to 15 g/L, preferably 5-7 g/L for complex medium media based on wet cell weight, wherein said culture medium has the following parameters: (a) pH in the range of 3.0 to 8.0, preferably 6.0 to 6.8 for complex medium, (b) temperature in the range of 25 to 45 degree C, preferably 37 to 42 degree C, and (c) dissolved oxygen: 20-80% of saturation, preferably 20-40% of saturation; and said culturing is carried out for a duration of 4 to 10 hours, preferably 6 hours.
The expression of recombinant OSMnat protein is induced after reaching appropriate biomass buildup using suitable lac operon inducer, such as lactose and the like, preferably IPTG at concentration of 0.1mM to 1mM, preferably 0.25mM.
The embodiments of the present invention comprise a process for isolating and purifying OSM from a OSM-producing microorganism, comprising the steps: a) lysing the microorganism and separating insoluble material comprising OSM from soluble proteinaceous cell material; b) solubilising the OSM present in the insoluble material; c) oxidizing the OSM in the presence of a pair oxidizing/reducing agent; d) subjecting the solution to chromatography; and e) recovering purified OSM.

Preferably the OSM is human recombinant methionylated oncostatin M (rmetHuOSM).

In one of preferred embodiments of the present invention the pair of oxidizing/reducing agent is a pair of oxidized/reduced glutathiones, wherein the molar ratio of oxidized and reduced glutathione is from 1:5 to 1:20, preferably 1 :20.
In preferred embodiment of the present invention in step c) the OSM in the insoluble material is solubilized using a chaotropic agent, preferably at an intermediate concentration of a chaotropic agent, at pH in the range 8.00-9.00.
The embodiment of the present invention further comprises separation of the refolded OSM from chaotrope, wherein the refolded OSM is separated from chaotrope by gel-filtration, preferably the gel-filtration column is Sephadex G-25.
The chromatography in step d) is a one-step chromatography purification, preferably one-step ion exchange chromatography.
The solubilising in step b) of present invention might be performed by using guanidinium hydrochloride, the concentration of guanidinium hydrochloride being from 3.0 to 7.0 , preferably 6.0 - 6.2 M.

Step d) of the process of the present invention is performed on CM-Sepharose or SP-Sepharose column, preferably at pH of from 5.2 to 5.6.
In one of the embodiment of the present invention a process for isolating and purifying rmetHuOSM from a OSM producing microorganism comprises: a) lysing the microorganism and separating insoluble material containing rmetHuOSM from soluble proteinaceous material; b) solubilizing the rmetHuOSM present in the insoluble material; c) oxidizing the rmetHuOSM using oxidized glutathione in the presence of reduced glutathione; d) separating of refolded rmetHuOSM from chaotropic agent e) one-step chromatography purification of rmetHuOSM.
One of the important objects is a preparation of purified OSM protein, obtained by the process of the present invention.

The invention is described below in further details and with reference to the accompanying drawings and examples.

### BRIEF DESCRIPTION OF THE DRAWINGS:

The preparation of human mature Oncostatin M protein according to present invention is illustrated by Figures 1-9.
Fig. 1 is the map of expression vector. The expression vector is plasmid pET21a+/OSM, carrying gene of oncostatin M, inserted via the target sites of Ndel and Hindlll restriction endonucleases.
Fig.2 shows the sequence and translation of rmetHuOSM gene into the polypeptide chain. Fig. 2 also shows the amino acid sequence of the protein.
Fig. 3 represents comparison of three E.coli strains, as shown in SDS-PAGE in Western blot. E.coli strains BL21(DE3), K802 and Rosetta gami-2 are presented, accordingly in 1-2, 3-4 and 5-6 lanes. First sample is soluble fraction of cell protein, second - insoluble fraction.
Fig. 4 represents E. coli Rosetta gami-2/pET21a+/OSM growth in 3 L fermenter in modified M9 medium. Operational on-line and off-line parameters are presented, such as oxygen saturation, pH, temperature, stirrer speed, airflow velocity and optical density.
Fig. 5 represents an image of SDS-PAGE after biosynthesis process, in first lane presenting a molecular weight marker (corresponding to values of Fig.3), 10 micrograms of soluble fraction was loaded on second lane, and 10 micrograms of insoluble cell fraction were loaded on third lane.
Fig. 6 represents the purity of processed according to this invention and formulated rmetHuOSM as judged by SDS-PAGE both under reducing conditions. First lane, a prestained protein marker from Thermo Fisher Scientific (Fermentas), with 10, 25, 35, 40, 55, 70, 100 and 130 kDa molecular standards, second to fourth lanes - purified OSM in reducing conditions, 1.25, 0.5 and 0.25 micrograms of sample loaded on lanes, accordingly.
Fig. 7 represents the purity of processed according to this invention and formulated rmetHuOSM as judged by reversed-phase HPLC.
Fig. 8 presents two mass specters from ESI-TOF mass spectrometer, as described in Example 7.
Fig. 9. Theoretical sequence comparison with acquired data. As it can be seen, N-terminal methionine is removed after purification process and only fully native sequence of OSM is retained in the solution of the final preparation.

### Detailed Description of the Invention

The present invention aims to provide a method of producing recombinant DNA which encodes a polypeptide displaying physicochemical properties of mature human Oncostatin (196) M. Another objective of the present invention is to produce OSM196 by controled fermentation. Yet another objective of the present invention is to obtain OSM protein in a pure form. A further object of the present invention is to prepare a solution composition comprising of the said recombinant human OSM protein.

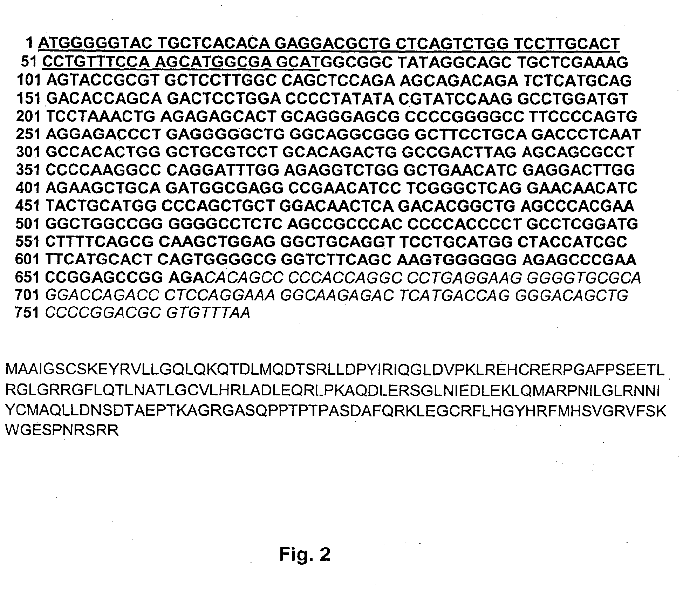

A pCMV shuttle carrying full open reading frame sequence with both pre- and propeptides was used as matrix for gene amplifying. The sequence is of cDNA origin. Accordingly, in one aspect, the present invention provides an isolated nucleic acid molecule having the nucleotide sequence set out in Fig.2. In another aspect there is provided an expression plasmid comprising a nucleic acid molecule having the nucleotide sequence set out in Fig. 2 (SEQ ID NO:1):

1 ATGGGGGTAC TGCTCACACA GAGGACGCTG CTCAGTCTGG TCCTTGCACT
51 CCTGTTTCCA AGCATGGCGA GCATGGCGGC TATAGGCAGC TGCTCGAAAG
101 AGTACCGCGT GCTCCTTGGC CAGCTCCAGA AGCAGACAGA TCTCATGCAG
151 GACACCAGCA GACTCCTGGA CCCCTATATA CGTATCCAAG GCCTGGATGT
201 TCCTAAACTG AGAGAGCACT GCAGGGAGCG CCCCGGGGCC TTCCCCAGTG
251 AGGAGACCCT GAGGGGGCTG GGCAGGCGGG GCTTCCTGCA GACCCTCAAT

301 GCCACACTGG GCTGCGTCCT GCACAGACTG GCCGACTTAG AGCAGCGCCT
351 CCCCAAGGCC CAGGATTTGG AGAGGTCTGG GCTGAACATC GAGGACTTGG
401 AGAAGCTGCA GATGGCGAGG CCGAACATCC TCGGGCTCAG GAACAACATC
451 TACTGCATGG CCCAGCTGCT GGACAACTCA GACACGGCTG AGCCCACGAA
501 GGCTGGCCGG GGGGCCTCTC AGCCGCCCAC CCCCACCCCT GCCTCGGATG
551 CTTTTCAGCG CAAGCTGGAG GGCTGCAGGT TCCTGCATGG CTACCATCGC
601 TTCATGCACT CAGTGGGGCG GGTCTTCAGC AAGTGGGGGG AGAGCCCGAA
651 CCGGAGCCGG AGACACAGCC CCCACCAGGC CCTGAGGAAG GGGGTGCGCA
701 GGACCAGACC CTCCAGGAAA GGCAAGAGAC TCATGACCAG GGGACAGCTG
751 CCCCGGACGC GTGTTTAA

OSM gene sequence was obtained from OriGene Technologies, Inc. in DNA shuttle pCMV6. The coding DNA sequence obtained in this vector is as presented in Fig. 2, reflecting the whole open reading frame of OSM gene, with its signal sequence on N-end of protein (here presented as underlined text) and pro sequence on C-end of protein (presented as italic text).

Gene sequence without its pre- and pro-sequences was designated as OSM(196) to refer to its amino acids sequence length. Gene codes for protein sequence presented here. N-terminal methionine is bacterial expression system artefact.

Suitably, the expression has a strong T7 promoter. Preferably, the expression plasmid is pET21a+/OSM

In another aspect there is provided a host cell comprising the expression plasmid in accordance with the invention. Suitably, the host cell is E.coli. In one embodiment, the E.coli is E.coli strain Rosetta gami-2, carrying additional pRARE plasmid coding for rare codon tRNAs. Rosetta gami-2 strain has also two mutations: trx and gor, allowing folding recombinant protein into a correct form. These mutations are further tuned in in lower than optimal cultivation temperatures.

In a further aspect, there is provided a primer sequences for amplification of a modified hOSM sequence. Accordingly, primer sequences are as follows:
FORWARD PRIMER: 5'-AAACATATGGCGGCTATAGGCAGCT-3' and
REVERSE PRIMER 5'-TTTAAGCTTCTATCTCCGGCTCCGGTT-3'.

Suitably, recombinant DNA from pCMV shuttle is amplified using recombinant DNA polymerase, such as Pfu or Taq. After amplification, PCR product is directly cloned into intermediate plasmid, or digested with restriction endonucleases Ndel and Hindlll and cloned into accordingly digested plasmid pET21a+. After transformation into intermediate E.coli strain, such as JM109 or RRI, clones are selected, verified and retransformed into expression strain.

Accordingly, biosynthesis of verified expression clone is performed using modified M9 medium or another complex medium. Fermentation process is performed in bioreactor, using output controlers such as pO₂ and pH electrodes, thermo pore or stirrer. Prior induction, cultivation temperature is elevated from +37°C to +42°C, if insoluble protein form is desired.

The present invention also provides a novel process for isolating and purifying rmetHuOSM from a OSM producing microorganism.

Accordingly, in this aspect of the invention there is provided a process for isolating and purifying OSM from a OSM-producing microorganism comprising the steps: a) lysing the microorganism and separating insoluble material comprising OSM from soluble proteinaceous material; b) solubilising the OSM present in the insoluble material; c) oxidizing the OSM in the presence of a pair oxidizing/reducing agent; d) subjecting the solution to chromatography; and e) recovering purified OSM.

Prior to lysis, the cells are harvested from the culture, and may be concentrated if necessary, by filtration, centrifugation, and other conventional methods. In a preferred embodiment of the present invention, the microorganism producing OSM is E. coli. Suitable strains of E. coli for production are known to the person skilled in the art.

In accordance with the procedures of the present invention, the cell membranes of the microorganisms are lysed using conventional techniques such as homogenization, sonication, or pressure cycling. Preferred methods include sonication or homogenization with a Rannie homogenizer.

After the cells have been lysed, the particulate matter containing rmetHuOSM is separated from the liquid phase of lysate and resuspended in appropriate buffer solution. The particulate matter may be optionally washed to remove any water soluble E. coli proteins therein.

Suitably, the rmetHuOSM in the particulate matter is solubilized in the presence of a solubilizing agent preferably above neutral pH conditions. The solubilizing agent is a chaotropic agent (i.e., a protein denaturant that dissociates hydrogen bonds and affects the tertiary structure of the proteins causing its unfolding) generally in an aqueous buffer solution.

Accordingly, in one embodiment, the OSM in the insoluble material is solubilized using a chaotropic agent. Representative chaotropic agents include urea and guanidinium hydrochloride. Guanidinium hydrochloride is a stronger chaotropic agent and is preferred avoiding carbamoylation of polypeptide chain which may occur if concentrated urea solution is used. Accordingly, in one embodiment, step b) includes incubation with guanidinium hydrochloride. The concentration of the chaotropic agent will depend upon the particular agent that is used and the amount of cellular material present. In one embodiment, in step b) the concentration of guanidinium hydrochloride is from 3.0 to 7 M and most preferably a 6M guanidinium hydrochloride solution is employed.

The pH may be adjusted by adding suitable buffers, and preferably the pH will range from about 7.0 to about 9.0 and most preferably within pH range 8.4 - 8.8.

Following solubilization of the rmetHuOSM, insoluble particulate matter is separated and discarded.

Suitably, the soluble rmetHuOSM is oxidized in the presence of a pair reducing/oxidizing agent, Suitable pair reducing/oxidizing agents include, for example, cysteine and cystine, dithiothreitol and its oxidized form, glutathione and oxidized glutathione [B. Fischer, I. Summer and P. Goodenough. Isolation, renaturation, and formation of disulfide bonds of eukaryotic proteins expressed in Escherichia coli as inclusion bodies. Biotechn. Bioengn.,41, 3-13 (1993); V. Lozanov, C. Guarnaccia, A. Patthy, S. Foot and S. Pongor. Synthesis and cystine/cysteine-catalyzed oxidative folding of the Amaranth α-amylase inhibitor. J.Peptide Res., 50, 65-72 (1997); Y-J. Li, D. M. Rothwarf, and H. A. Scheraga. An unusual adduct of dithiothreithol with a pair of cysteine residues of a protein as a stable folding intermediate. J. Amer. Chem. Soc, 120, 2668-2669 (1998)], Advantageously, it has been found that the yield of correctly folded rmetHuOSM, that is, oxidized rmetHuOSM having the correct native conformation of disulfide bonds, is increased by facilitating rearrangement of disulfide bonds through the use of a glutathione redox buffer (glutathione and oxidized glutathione). Accordingly, in one embodiment, the pair reducing/oxidizing agent used in step c) is a glutathione redox buffer (glutathione and oxidized glutathione).

The rmetHuOSM is oxidized by the oxidized glutathione and the presence of the reducing agent, glutathione, in the redox buffer substantially reduces the formation of incorrectly folded rmetHuOSM, that is rmetHuOSM with incorrect disulfide bonds. The ratio of glutathione: oxidized glutathione in the redox buffer is readily ascertained by one of ordinary skill in the art. Preferably an excess of glutathione is employed, more preferably a ratio of from 5:1 to 20:1 on a molecular weight basis glutathione: oxidized glutathione is employed. Most preferably a 20:1 molar ratio of glutathione: oxidized glutathione is employed.

Suitably, the refolding of rmetHuOSM in the presence of redox buffer containing a pair reduced/oxidized glutathione is performed at an intermediate concentration of a chaotropic agent. In one embodiment, the refolding is at an intermediate guanidinium hydrochloride concentration. Suitably, the concentration of guanidinium hydrochloride is from 3M to 7M and most preferably in buffer solution containing from 6.0 to 6.2M of guanidinium hydrochloride.

Yield of rmetHuOSM refolding is strongly dependent on pH value of refolding buffer containing intermediate concentration of guanidinium hydrochloride and redox system.

Preferably the refolding reaction is performed by maintaining the pH of buffer solution from 7.0 to pH 9.8 and the most preferably from pH 8.4 to pH 8.8. The resulting solution containing correctly folded rmetHuOSM is preferably centrifuged or filtrated to remove any remaining particulate matter and the resulting discarded solution is buffer exchanged to remove residual chaotropic agent such as guanidinium hydrochloride and constituents of redox system such as glutathione and oxidized glutathione.

Accordingly, in a further embodiment, the process of the invention additionally comprises separating the refolded OSM from chaotrope. Suitably, where the chaotropic agent is guanidinium hydrochloride, it may be removed from a solution containing refolded rmetHuOSM by gel-filtration. Suitable gel-filtration media will be familiar to those skilled in the art. In one embodiment, the gel-filtration media is Sephadex G-25 chromatography media.

Suitably, the pH of the buffer solution is adjusted to a pH range suitable for gel-filtration.

Where the gel-filtration media is Sephadex G-25, the pH is suitably adjusted to be between about 5.2 to 5.6 and preferably to 5.4. The resulting mixture is filtered and the collected filtrate exposed for maturation. Suitably, the collected filtrate is exposed for about from 20 to 90 hours for correctly folded rmetHuOSM maturation. Correctly folded rmetHuOSM is then separated from any remaining contaminants employing chromatographic procedures.

It is preferred to employ ion exchange liquid chromatography to recover the purified rmetHuOSM. Suitable methods for ion exchange liquid chromatography will be familiar to those skilled in the art. In one embodiment of the process of the invention, the chromatography in step d) is a one-step chromatography purification. Suitably, the chromatography is one-step ion exchange chromatography.

In a preferred mode of practice of this aspect of the invention, high yields of purified rmetHuOSM are recovered through the use of a CM-Sepharose or SP Sepharose ion exchange column which preferably operates at pH of a buffer of chromatography from 5.2 to 5.6, and most preferably at pH about 5.4.

Accordingly, in a preferred embodiment, the present invention provides a process for isolating and purifying rmetHuOSM from a OSM producing microorganism comprising: 1) lysing the microorganism and separating insoluble material containing rmetHuOSM from soluble proteinaceous material; 2) solubilizing the rmetHuOSM present in the insoluble material; 3) oxidizing the rmetHuOSM using oxidized glutathione in the presence of reduced glutathione; 4) separating of refolded rmetHuOSM from chaotrope 5) one-step chromatography purification of rmetHuOSM.

In another embodiment, the invention provides a process comprising the steps of: 1) lysing the microorganism and separating insoluble material containing rmetHuOSM from soluble proteinaceous material; 2) solubilizing the rmetHuOSM present in the insoluble material; 3) oxidizing the rmetHuOSM in the presence of a pair of reducing and oxidizing agent; 4) separating of rmetHuOSM from solubilizing agent; 5) time-dependent maturation of rmetHuOSM; 6) selectively separating correctly folded rmetHuOSM from incorrectly folded, aggregated and altered rmetHuOSM by cation-exchange chromatography; 7) transition of highly purified, correctly folded rmetHuOSM to its stable liquid formulation by chromatography over gel-filtration column.

### MODES FOR CARRYING OUT THE INVENTION

### Examples of embodiments of the invention

In the following examples, Escherichia coli strain JM109 was used as transient host strain, for transformation with the vector, E.coli Rosetta gami-2 strain was used as expression strain. The transformed E.coli host of present invention contained an expression vector prepared from pET21a+ (Novagen). This vector was ligated with amplified truncated DNA sequence of native OSM after double restriction digestion with Ndel+Hindlll. The fermentation process involved applying approaches to obtain high expression cell culture described in this document. The OSM protein is accumulated in inclusion bodies and purified using simple techniques as described elsewhere in the document.

Various modifications and variations of the described methods and system of the present invention will be apparent to those skilled in the art without departing from the scope and spirit of the present invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### EXAMPLE 1. AMPLIFICATION OF MATURE OSM GENE

OSM gene preparation for fusion consisted of two step process: PCR optimization and gene amplification with PCR, adding Ndel site on its 5' end and Hindlll on its 3' end. Primers were: FORWARD PRIMER: 5'-AAACATATGGCGGCTATAGGCAGCT-3' and REVERSE PRIMER 5'-TTTAAGCTTCTATCTCCGGCTCCGGTT-3'. The sequence of forward and reverse primers is given in 5' to 3' direction. PCR optimization for OSM gene was performed in order to receive a specifically amplified PCR product in desirable concentration interval. Optimisation parameters included MgSO₄ concentration (1 - 4 mM), primers concentration (0.1 - 1µM) and annealing temperature (58 - 72°C), thus forming a net experiment of 48 different reaction conditions. DNA polymerase used for amplification was Pfu (recombinant, Fermentas, Thermo Fisher Scientific) due its proof-reading feature, good processivity and low error rate. For OSM PCR product (609 bp length), the best conditions were as follows: 2 mM of MgSO₄, 0.1 µM of each primer, and program with two-step annealing step:

**Table 1**

| Cycle No | Step | Temperature | Time |
|---|---|---|---|
| | Initial denaturation | 95 °C | 10 min. |
| 20 | Matrix DNA denaturation | 95 °C | 30 s |
| | Primer annealing | 59,8 °C | 45 s |
| | DNA synthesis | 72 °C | 80 s |
| 25 | Matrix DNA denaturation | 95 °C | 30 s |
| | Primer annealing | 72 °C | 80 s |
| | DNA synthesis | 72 °C | 10 min. |

The PCR product was attainable in 100-120 ng/µl concentration range

### EXAMPLE 2. OSM GENE CLONING INTO PET21A+ PLASMID

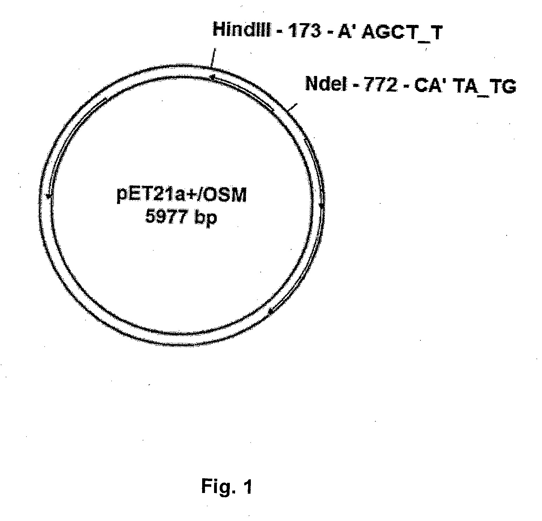

The obtained PCR product is purified using Fermentas GeneJet PCR purification kit and digested using two restriction endonucleases - Ndel and Hindlll. Digestion is performed overnight at +37°C temperature. After digestion, DNA fragment is repeatedly purified using the kit, removing small nucleotide fragments and restrictases. pET21+a plasmid (Novagen) is digested and purified likewise, but purification kit used for long DNA fragment is GenJet plasmid purification kit. Sticky ends of plasmid are dephosphorylated using CIAP enzyme, which is inactivated prior ligation. Gene and plasmid are ligated in molecular ratio of 10:1 for 16 hours in +4°C temperature using T4 DNA ligase (5 units per reaction mixture). Ligation mixture is transformed into chemically competent E. coli JM109 strain. After right-size insert clone selection, a selected positive clone is transformed into expression strain of E.coii Rosetta gami-2. pET21a+/OSM clone is cultivated, structure of plasmid DNA is confirmed using restriction analysis pattern. The map of plasmid is presented on Figure 1, alongside with typical electrophoresis pattern.

pET21a+/OSM plasmid was also cloned for comparison into E.coli strains BL21(DE3) and K802. Positive clones were verified by restriction analysis and metabolite consumption.

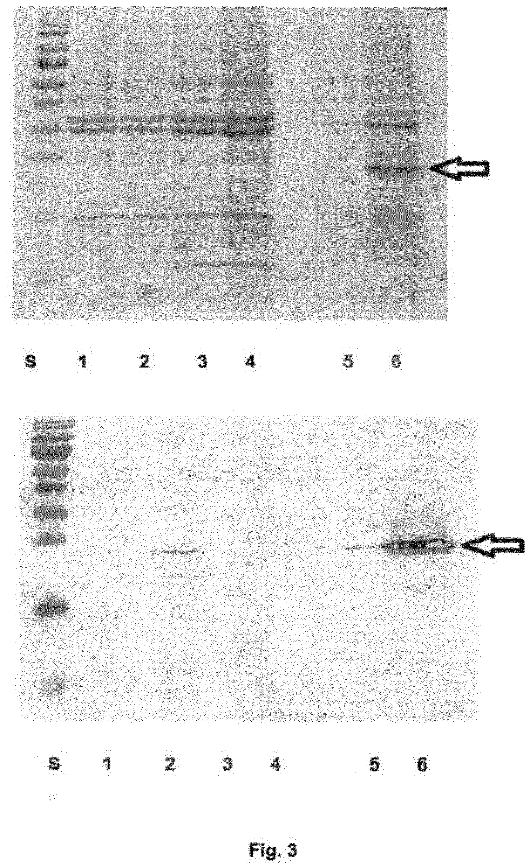

Fig. 3 represents comparison of three E.coli strains, as shown in SDS-PAGE and Western blot. E.coli strains BL21(DE3), K802 and Rosetta gami-2 are presented, accordingly in 1-2, 3-4 and 5-6 lanes. First sample is soluble fraction of cell protein, second - insoluble fraction. As it can be seen, only E.coli Rosetta gami-2 strain is high-producing clone.

### EXAMPLE 3. PREPARATION OF RESEARCH MASTER CELL BANK

After primary characterization, Rosetta gami-2 pET21a+/OSM clone was seeded into LB medium into 150ml flask and cultivated overnight at +37°C. After 16 hours, culture was reseeded into 150ml of LB medium with final optical density of 0.1 A.U. and further cultivated in orbital microbiological shaker for four hours at +37°C. Final optical density before conservation was 3.4 A.U. A final concentration of 10% of sterile glycerol was added to microbial culture and aliquoted into vials by 1 ml volume. Stock was freezed and stored in -75°C temperature.

### EXAMPLE 4. BIOSYNTHESIS PROCESS DESCRIPTION

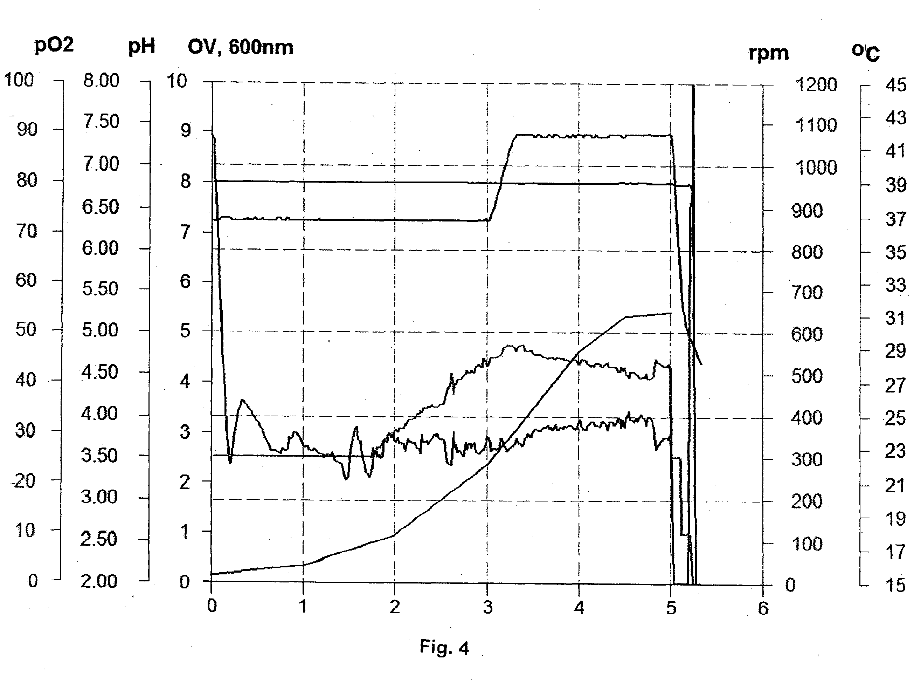

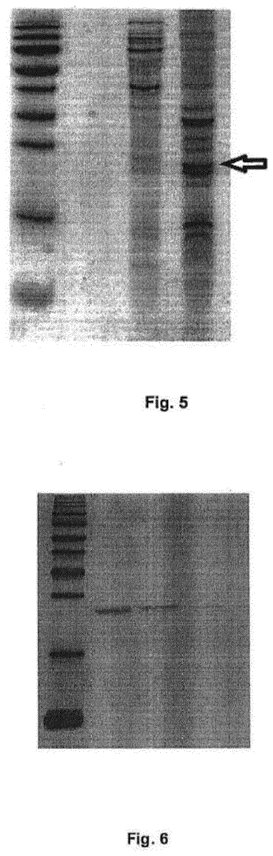

In 7 L fermenter, 4.7 L of complex medium was prepared, sterilized and inoculated with the inoculum to final optical density of 0.2 A.U. The agitation rate varied between 200 to 900 rpm. The aeration rates varied from about 0.3 L/min airflow of air supplied. M9m medium (for fermentation) composition (g/L): yeast extract - 20.0, ammonium chloride - 5.0, magnesium sulfate heptahydrate - 0.5, di-sodium hydrogen phosphate - 4.7, potassium dihydrogen phosphate - 4.5, glucose monohydrate - 22; supplemented with 50 ng/L of ampicillin and 34 ng/L of chloramphenicol. The air supplied was mixed with sufficient oxygen whenever required, in order to maintain dissolved oxygen at about 20 to 60% saturation. The fermentation was carried out at 37 degree C for 3 hours. After 3 hours, an induction is started with 0.25 mM final concentration of IPTG solution and the temperature is elevated to 42° C. After 2 hours of induction, cell material is collected by centrifuging culture medium at 8000 rpm for 30 minutes at 4° C. Typical biosynthesis batch record is presented in Fig. 4. In this figure E. coli Rosetta gami-2/pET21a+/OSM growth in 3 L fermenter operational on-line and off-line parameters are presented, such as oxygen saturation, pH, temperature, stirrer speed, airflow velocity and optical density. Fig.5 represents an image of SDS-PAGE after biosynthesis process, in first lane presenting a molecular weight marker, 10 micrograms of soluble fraction was loaded on second lane, and 10 micrograms of insoluble cell fraction were loaded on third lane.

### EXAMPLE 5. DESCRIPTION OF THE DOWNSTREAM PROCESS

Cell paste containing rmetHuOSM in transformed E. coli cells, such as obtained from Example 4, was dispersed in tank with agitator in 10 parts 0.1 M Tris buffer (pH 7.50, containing 2 mM EDTA, 1mM PMSF, 5mM DTT) at a temperature of approximately 5 DEG C. The suspension was passed through a Rannie high pressure homogenizer two times or sonicated by 3min for 1 g of biomass, The homogenate was centrifuged at a temperature of approximately 5 DEG C. The pellets were diluted with 10 parts 0.025 M Tris buffer, pH 8.0, containing 0.5 M NaCl, 0.1% Polysorbate-80, mixed with IKA mixer and the resulting mixture was centrifuged at a temperature of 5 DEG C. The supernatant was decanted and the remaining residue was resuspended with IKA mixer in 0.025 M Tris buffer (pH 7.00, containing 1.3 M Urea) to yield a mixture having a final volume of 10 parts water. The supernatant was decanted and the remaining residue was resuspended with IKA mixer in 0.025 M Tris buffer (pH 8.00, containing 0.6 M Urea) to yield a mixture having a final volume of 10 parts. The resulting mixture was centrifuged at a temperature of 5° C and the supernatant was decanted and the remaining residue was suspended with IKA mixer in 0.025 M Tris buffer (pH 8.50, containing 0.5M NaCl) to yield a mixture having a final volume of 10 parts water. The resulting mixture was centrifuged at a temperature of 5° C and the supernatant was decanted. The resulting residue was suspended with IKA homogenizer in 6 parts of 50 mM Glycine/NaOH buffer, pH 8.7, containing 6M GdmHCl; 0.5 M NaCl and 20mM DTT for 2-18 hours at a temperature of 5° C.

The resulting mixture was centrifuged at a temperature of 5°C and the supernatant was collected. The supernant was drop wise added into refolding solution (25 mM Glycine/NaOH buffer, pH 8.7, containing for final concentration 4mM GSH, 0.2mM GSSG, 0.5M L-Arginine) in ratio 1:3. The resulting mixture was adjusted to pH 8.70 and maintained at approximately 5° C for 60 hours. The solution was loaded onto a Sephadex G-25 at 5 ° C and eluted with 20 mM Sodium acetate (pH 5.30, containing 50 mM NaCl). The eluate loaded on to a CM-Sepharose column at 25° C. Biologically active rmetHuOSM was eluted from the column with a linear gradient from 20 mM Sodium acetate, 50 mM NaCl (pH 5.3) to 20 mM Sodium acetate, 400 mM NaCl (pH 5.3). The eluate containing highly purified rmetHuOSM was diluted with buffer to a final concentration of 20mM Na acetate, 250mM NaCl, pH 5.3. Fig. 6 represents the purity of processed protein according to this invention as judged by SDS-PAGE both under reducing conditions. First lane, a prestained protein marker from Thermo Fisher Scientific (Fermentas), with 10, 25, 35, 40, 55, 70, 100 and 130 kDa molecular standards, second to fourth lanes - purified OSM in reducing conditions, 1.25, 0.5 and 0.25 micrograms of sample loaded on lanes, accordingly.

### EXAMPLE 6. RP-HPLC ANALYSIS

RP-HPLC method was used for evaluation of OSM protein purity. This method was used to test the purity of OSM during purification steps and in final product solution. The experimental details are discribed below.

### Experimental details:

Mobile phase: A - 70% water, 30% acetonitrile and 0.2% TFA; B - 20% water, 80% acetonitrile and 0.2% TFA
Column: Symmetry 300 C18, 4.6 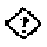 250 mm, particle size 5 mµ.
Chromatography system: HPLC system Alliance, Waters
Wavelength of detection: 280 nm
Flow rate: 1ml/min
RP-HPLC instrument method

**Table 2**

| No. | Flow, | Time, | Mobile | Notes | |
|---|---|---|---|---|---|
| | | | A, % | B, % | |
| 1 | 1.0 | 0 | 80 | 20 | Initial |
| 2 | 1.0 | 1 | 80 | 20 | Isocratic |
| 3 | 1.0 | 20 | 64 | 36 | Gradient |
| 4 | 1.0 | 21 | 10 | 90 | Gradient |
| 5 | 1.0 | 22 | 10 | 90 | Isocratic |
| 6 | 1.0 | 23 | 80 | 20 | Gradient |
| 7 | 1.0 | 27 | 80 | 20 | Isocratic |

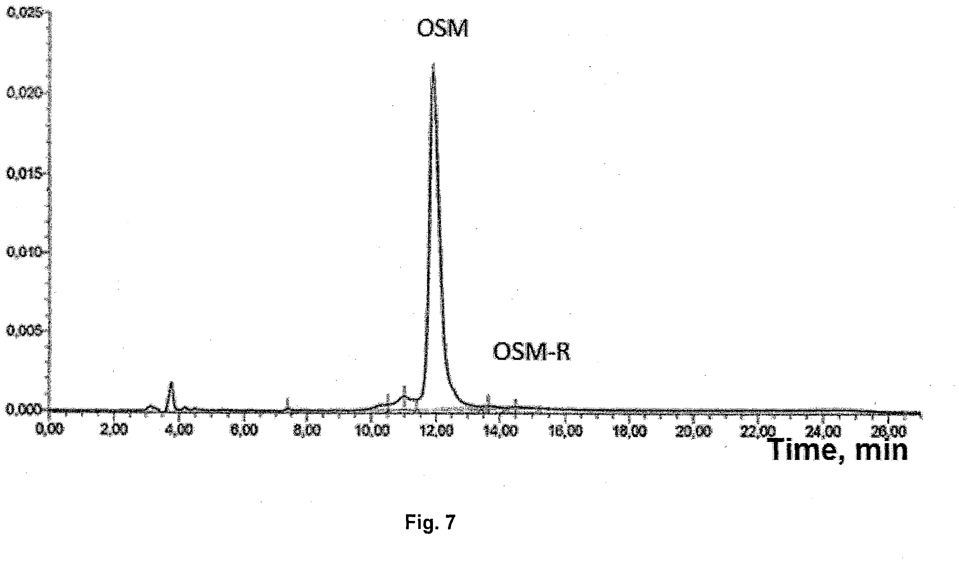

Fig.7 represents the purity of processed according to this invention and formulated rmetHuOSM as judged by reversed-phase HPLC. As can be seen from the integration of the peak area, the purity of purified protein is up to 91.2% with 0.5% of the reduced form thereof.

### EXAMPLE 7. ONCOSTATIN M MOLECULAR MASS DETERMINATION USING MASS SPECTROMETER.

For purified protein identification, molecular weight measurement and partial sequencing of OSM was performed by mass spectrometry methods. Equipment used was MicrOTOF q2 with ESI source, Bruker. Materials: acetonitrile, formic acid, PD MiniTrap G-25 columns, 5mM Ammonium bicarbonate buffer.

Sample preparation. Sample buffer is exchanged to 5mM Ammonium bicarbonate buffer. This is done using PD MiniTrap G-25 according to manufacturer's instructions. If needed sample solution is concentrated to 1mg/ml. Before subjecting sample to MS analysis, it is diluted 1:1 (v:v) with Acetonitrile, 0.2% Formic acid solution to final concentration of 0.5mg/ml. Mass spectrometer settings:

**Table 3**

| Parameter name | Value |
|---|---|
| Nebulizer | 0.3 bar |
| Dry gas | 4.0 l/min |
| Dry temperature | 180 °C |
| Mass range | 50-3000m/z |

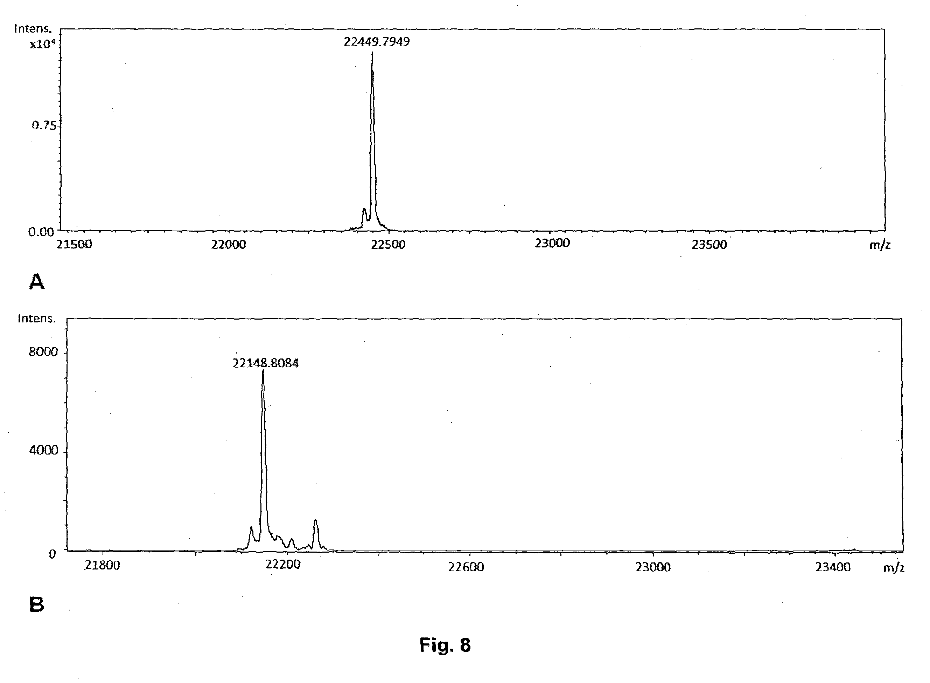

Mass spectra is acquired for 5min., then averaged and deconvoluted using Maximum Entropy algorithm (see Fig. 8). Figure 8 presents two mass spectra from ESI-TOF mass spectrometer. Measured mass of Oncostatin M is 22449Da (as observed in Figure 7, part A). Measured mass differs from theoretical by ∼300Da. This is because of a glutathione molecule that is bonded to Cys80 residue. After protein reduction glutathione molecule is removed, as seen in Fig.8 , part B (mass detected equals 22149 Da, which is the molecular weight of native protein).

Oncostatin M sequence analysis using Maldi-Tof mass spectrometer was performed using Autoflex speed, Bruker. Materials were as follows acetonitrile, triflouracetic acid, PD MiniTrap G-25 columns, 25mM Ammonium bicarbonate buffer and α-cyano-4-hydroxycinnamic acid.

Sample preparation. Sample buffer is exchanged to 25mM ammonium bicarbonate buffer. This is done using PD MiniTrap G-25 according to manufacturer's instructions. Trypsin is added with ratio 1:50 (Protease : Protein) and this solution is incubated in 37°C temperature for 12 hours. Sample is cleaned with ZipTip (according to manufacturer's instructions), mixed in equal volumes with α-cyano-4-hydroxycinnamic acid solubilized in 50% acetonitrile 0.1% triflouracetic acid. Prepared sample (0,5-1µl) is carried on to stainless steel Maldi plate, droplets left to fully dry. Mass spectrometer settings:

**Table 4**

| Parameter name | Value |
|---|---|
| Ion Source 1 | 19,6kV |
| Ion Source 2 | 16,7KV |
| Lens | 7,8kV |
| Reflector | 21,1 kV |
| Matrix suppression | up to 430Da |

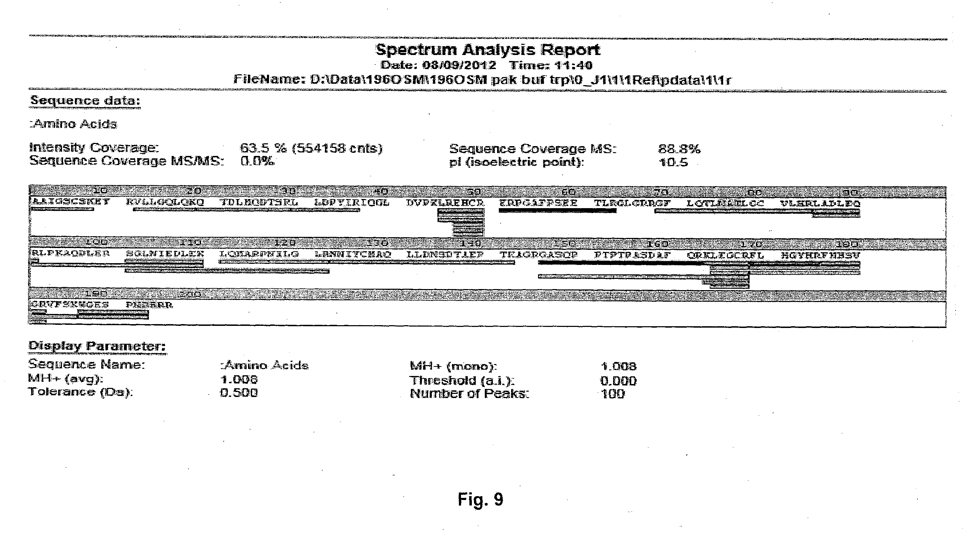

500-1500 shots of laser is applied to each sample spot. Peptides resulting from Oncostatin M digestion are analyzed and compared with theoretical peptide masses. Theoretical sequence comparison with acquired data is presented in Fig. 9. As it can be seen, N-terminal methionine is removed after purification process and only fully native sequence of OSM is retained. Partial sequencing results confirm that purified protein is 196 aa Oncostatin M and that it does not have methionine residue at N-termini. 89% of sequence is covered.

### INDUSTRIAL APPLICABILITY

The Oncostatin M protein and stable preparations thereof, obtainable according to present invention, are useful as antiviral and antitumor agent. Oncostatin M (OSM) was initially identified as a polypeptide cytokine which inhibited the in vitro growth of cells from melanoma and other solid tumors. OSM shows significant similarities in primary amino acid sequence and predicted secondary structure to leukemia inhibitory factor (LIF), ciliary neurotrophic factor (CNTF), granulocyte colony-stimulating factor (G-CSF), interleukin 6 (IL-6), and interleukin 11 (IL-11). Recent data indicates that OSM also shares a number of in vitro activities with other members of this cytokine family. The 196 residue OSM is the predominant form isolated form a variety of cell lines and corresponds to a glycoprotein of 28 KDa, although the larger 227 residue pro-OSM can be isolated from over transfected cells.

## Claims

1. A process for preparation and purification of recombinant human oncostatin M protein (OSMnat), comprising:
i) cultivating recombinant Escherichia coli containing an OSMnat gene,
ii) culturing said recombinant Escherichia coli in complex culture medium to produce OSMnat protein, and
iii) isolating and purifying recombinant OSMnat protein from inclusion bodies or soluble protein fraction,
wherein said recombinant Escherichia coli containing an OSMnat gene is cultivated by amplifying said gene and cloning said amplified modified OSMnat gene into an intermediate or expression vector, amplifying and isolating said OSMnat gene from said vector cloned and transforming said expression vector into said Escherichia coli,
wherein said OSMnat gene comprises SEQ ID NO:1, said Escherichia coli is Escherichia coli Rosetta gami-2, and said vector is pET21a+, and
wherein isolating and purifying of recombinant OSMnat protein obtained comprises the steps of:
a) lysing the microorganism and separating insoluble material comprising OSM from soluble proteinaceous cell material;
b) solubilising the OSM present in the insoluble material;
c) oxidizing the OSM in the presence of a pair oxidizing/reducing agent, wherein the pair of oxidizing/reducing agent is a pair of oxidized/reduced glutathiones, wherein the molar ratio of oxidized and reduced glutathione is 1:20 ;
d) subjecting the solution to chromatography; and
e) recovering purified OSM.

2. The process according to claim 1, wherein said culture medium is complex medium, selected from LB, YPD, TB, M9 or M9m, preferably M9m, wherein said culture medium comprises one or more nitrogen sources, selected from the group consisting of ammonium salts, nitrates, tryptone, yeast extract and ammonium hydroxide, and a carbon source, selected from the group consisting of glycerol, glucose, fructose, preferably glucose.

3. The process according to claim 1, wherein said Escherichia coli Rosetta gami-2 is carrying additional pRARE plasmid.

4. The process according to any of claims 1 to 3, wherein said OSMnat gene is cloned in pET21a+ vector downstream to early bacteriophage T7 promoter, said gene being in a reading frame from promoter, lac operon being upstream from T7 promoter, and the expression of desired construct, containing a OSMnat gene, is stringently controlled and terminated by T7 terminator.

5. The process according to any of preceding claims, wherein said Escherichia coli has resistance to antibiotics respectably accordingly host strain, such as ampicillin, kanamycin, chloramphenicol and tetracycline.

6. The process according to any of preceding claims, wherein the biomass build up is in a range of 2 to 15 g/L, preferably 5-7 g/L for complex medium media based on wet cell weight, wherein said culture medium has the following parameters: (a) pH in the range of 3.0 to 8.0, preferably 6.0 to 6.8 for complex medium, (b) temperature in the range of 37 to 42 degrees C, and (c) dissolved oxygen: 20-80% of saturation, preferably 20-40% of saturation; and said culturing is carried out for a duration of 4 to 10 hours, preferably 6 hours.

7. The process according to any of preceding claims, wherein the expression of recombinant OSMnat protein is induced after reaching appropriate biomass buildup using suitable lac operon inducer IPTG at concentration of 0.1mM to 1mM, preferably 0,25mM.

8. The process according to any of preceding claims, wherein the OSM in steps a) - c) is human recombinant methionylated oncostatin M (rmetHuOSM).

9. The process according to any of preceding claims, wherein in step b) the OSM in the insoluble material is solubilized using a chaotropic agent, preferably at an intermediate concentration of a chaotropic agent, at pH in the range 8,00-9,00.

10. The process according to claim 9, further comprising separating the refolded OSM from chaotrope, wherein the refolded OSM is separated from chaotrope by gel-filtration, wherein the gel-filtration column is preferably Sephadex G-25.

11. The process according to any of preceding claims, wherein the solubilising in step b) is using guanidinium hydrochloride, the concentration of guanidinium hydrochloride being from 3.0 to 7.0, preferably 6.0 M.

12. The process according to any of preceding claims, wherein the chromatography in step d) is an one-step ion exchange chromatography performed on CM-Sepharose or SP-Sepharose column at pH of from 5.2 to 5.6.

13. The process according to any of preceding claims, wherein purified OSM protein obtained is fully native OSM of 196 amino acids.

## Patentansprüche

1. Verfahren zur Präparation und Aufreinigung von rekombinantem menschlichem Oncostatin-M-Protein (OSMnat), umfassend:
i) Kultivieren rekombinanter Escherichia coli, die ein OSMnat-Gen enthalten,
ii) Kultivieren der rekombinanten Escherichia coli in komplexem Kulturmedium, so dass OSMnat-Protein produziert wird, und
iii) Isolieren und Aufreinigen von rekombinantem OSMnat-Protein aus Einschlusskörperchen oder löslicher Proteinfraktion,
wobei die Escherichia coli, die ein OSMnat-Gen enthalten, kultiviert werden, indem das Gen amplifiziert und das amplifizierte modifizierte OSMnat-Gen in einen Zwischen- oder Expressionsvektor kloniert wird, das OSMnat-Gen von dem klonierten Vektor amplifiziert und isoliert und der Expressionsvektor in die Escherichia coli transformiert wird,
wobei das OSMnat-Gen SEQ ID NO:1 umfasst, es sich bei den Escherichia coli um Escherichia coli Rosetta gami-2 und bei dem Vektor um pET21a+ handelt und
wobei Isolieren und Aufreinigen von erhaltenem rekombinantem OSMnat-Protein die folgenden Schritte umfasst:
a) Lysieren des Mikroorganismus und Trennen von OSM umfassendem unlöslichem Material von löslichem proteinhaltigem Zellmaterial;
b) Solubilisieren des im unlöslichen Material vorhandenen OSM;
c) Oxidieren des OSM in Gegenwart eines Paars Oxidations-/Reduktionsmittel, wobei es sich bei dem Paar Oxidations-/Reduktionsmittel um ein Paar oxidierte/reduzierte Glutathione handelt, wobei das Molverhältnis von oxidiertem und reduziertem Glutathion bei 1:20 liegt;
d) Durchführen einer Chromatographie mit der Lösung; und
e) Gewinnen von aufgereinigtem OSM.

2. Verfahren nach Anspruch 1, wobei es sich bei dem Kulturmedium um komplexes Medium ausgewählt aus LB, YPD, TB, M9 oder M9m, vorzugsweise M9m, handelt, wobei das Kulturmedium eine oder mehrere Stickstoffquellen, ausgewählt aus der Gruppe bestehend aus Ammoniumsalzen, Nitraten, Trypton, Hefeextrakt und Ammoniumhydroxid, und eine Kohlenstoffquelle, ausgewählt aus der Gruppe bestehend aus Glycerin, Glucose, Fructose und dergleichen, vorzugsweise Glucose, umfasst.

3. Verfahren nach Anspruch 1, wobei die Escherichia coli Rosetta gami-2 zusätzliches pRARE-Plasmid tragen.

4. Verfahren nach einem der Ansprüche 1 bis 3, wobei das OSMnat-Gen in Vektor pET21a+ stromabwärts vom frühen Bakteriophage-T7-Promotor kloniert ist, wobei das Gen in einem Leseraster vom Promotor liegt, das lac-Operon stromaufwärts vom T7-Promotor liegt, und die Expression von gewünschtem Konstrukt, das ein OSMnat-Gen enthält, unter stringenter Kontrolle und Termination durch T7-Terminator steht.

5. Verfahren nach einem der vorhergehenden Ansprüche, wobei die Escherichia coli Resistenz gegen Antibiotika respektabel gemäß dem Wirtsstamm, wie z. B. Ampicillin, Kanamycin, Chloramphenicol und Tetracyclin, aufweisen.

6. Verfahren nach einem der vorhergehenden Ansprüche, wobei die Anhäufung von Biomasse in einem Bereich von 2 bis 15 g/l, vorzugsweise 5-7 g/l für komplexes Medium bezogen auf das Zellfeuchtgewicht liegt, wobei das Kulturmedium die folgenden Kenngrößen aufweist: (a) pH-Wert im Bereich von 3,0 bis 8,0, vorzugsweise 6,0 bis 6,8 für komplexes Medium, (b) Temperatur im Bereich von 37 bis 42 Grad C und (c) gelöster Sauerstoff: 20-80% Sättigung, vorzugsweise 20-40% Sättigung; und das Kultivieren über eine Zeitdauer von 4 bis 10 Stunden, vorzugsweise 6 Stunden, erfolgt.

7. Verfahren nach einem der vorhergehenden Ansprüche, wobei die Expression von rekombinantem OSMnat-Protein nach Erreichen einer angemessenen Biomasseanhäufung induziert wird, wobei als geeigneter lac-Operon-Induktor IPTG in einer Konzentration von 0,1 mM bis 1 mM, vorzugsweise 0,25 mM, verwendet wird.

8. Verfahren nach einem der vorhergehenden Ansprüche, wobei es sich bei dem OSM in Schritt a) - c) um menschliches rekombinantes methionyliertes Oncostatin M (rmetHuOSM) handelt.

9. Verfahren nach einem der vorhergehenden Ansprüche, wobei in Schritt b) das OSM im unlöslichen Material unter Verwendung eines chaotropen Mittels, vorzugsweise bei einer mittleren Konzentration eines chaotropen Mittels, bei einem pH-Wert im Bereich von 8,00-9,00 solubilisiert wird.

10. Verfahren nach Anspruch 9, ferner umfassend Trennen des rückgefalteten OSM vom Chaotrop, wobei das rückgefaltete OSM vom Chaotrop durch Gel-filtration getrennt wird, wobei es sich bei der Gelfiltrationssäule vorzugsweise um Sephadex G-25 handelt.

11. Verfahren nach einem der vorhergehenden Ansprüche, wobei das Solubilisieren in Schritt b) unter Verwendung von Guanidiniumhydrochlorid erfolgt, wobei die Konzentration von Guanidiniumhydrochlorid bei 3,0 bis 7,0, vorzugsweise 6,0 M liegt.

12. Verfahren nach einem der vorhergehenden Ansprüche, wobei es sich bei der Chromatographie in Schritt d) um eine Ein-Schritt-Ionenaustauschchromatographie handelt, die an einer CM-Sepharose- oder SP-Sepharose-Säule bei einem pH-Wert von 5,2 bis 5,6 durchgeführt wird.

13. Verfahren nach einem der vorhergehenden Ansprüche, wobei es sich bei dem erhaltenen aufgereinigten OSM-Protein um vollnatives OSM mit 196 Aminosäuren handelt.

## Revendications

1. Procédé de préparation et de purification de protéine oncostatine M (OSMnat) humaine recombinante, comprenant :
i) la culture d'Escherichia coli recombinant contenant un gène OSMnat,
ii) la culture dudit Escherichia coli recombinant dans un milieu de culture complexe pour produire la protéine OSMnat, et
iii) l'isolement et la purification de protéine OSMnat recombinante à partir de corps d'inclusion ou de fraction de protéines solubles,
dans lequel ledit Escherichia coli recombinant contenant un gène OSMnat est cultivé par amplification dudit gène et clonage dudit gène OSMnat modifié amplifié dans un vecteur intermédiaire ou d'expression, amplification et isolement dudit gène OSMnat à partir dudit vecteur cloné et transformation dudit vecteur d'expression dans ledit Escherichia coli,
dans lequel ledit gène OSMnat comprend SEQ ID NO: 1, ledit Escherichia coli est Escherichia coli Rosetta gami-2, et ledit vecteur est pET21a+, et
dans lequel l'isolement et la purification de la protéine OSMnat recombinante obtenue comprend les étapes de :
a) lyse du micro-organisme et séparation du matériau insoluble comprenant OSM à partir d'un matériau cellulaire protéinique soluble ;
b) solubilisation de l'OSM présente dans le matériau insoluble ;
c) oxydation de l'OSM en présence d'une paire d'agents oxydant/réducteur, dans lequel la paire d'agents oxydant/réducteur est une paire de glutathions oxydé/réduit, dans lequel le rapport molaire de glutathion oxydé et réduit est 1:20 ;
d) soumission de la solution à une chromatographie ; et
e) récupération d'OSM purifiée.

2. Procédé selon la revendication 1, dans lequel ledit milieu de culture est un milieu complexe, choisi parmi LB, YPD, TB, M9 ou M9m, de préférence M9m, dans lequel ledit milieu de culture comprend une ou plusieurs sources d'azote, choisies dans le groupe constitué de sels d'ammonium, nitrates, tryptone, extrait de levure et hydroxyde d'ammonium, et une source de carbone, choisie dans le groupe constitué du glycérol, du glucose, du fructose, de préférence le glucose.

3. Procédé selon la revendication 1, dans lequel ledit Escherichia coli Rosetta gami-2 comporte un plasmide pRARE supplémentaire.

4. Procédé selon l'une quelconque des revendications 1 à 3, dans lequel ledit gène OSMnat est cloné dans le vecteur pET21a+ en aval du promoteur précoce de bactériophage T7, ledit gène étant dans un cadre de lecture à partir du promoteur, l'opéron lac étant en amont du promoteur T7, et l'expression de la construction souhaitée, contenant un gène OSMnat, est contrôlée de façon stringente et terminée par un terminateur T7.

5. Procédé selon l'une quelconque des revendications précédentes, dans lequel ledit Escherichia coli a une résistance aux antibiotiques, respectivement en correspondance à la souche hôte, tels que l'ampicilline, la kanamycine, le chloramphénicol et la tétracycline.

6. Procédé selon l'une quelconque des revendications précédentes, dans lequel l'accumulation de biomasse est dans une plage de 2 à 15 g/l, de préférence 5 à 7 g/l pour des milieux complexes sur la base du poids de cellules humides, dans lequel ledit milieu de culture a les paramètres suivants : (a) pH dans la plage de 3,0 à 8,0, de préférence 6,0 à 6,8 pour un milieu complexe, (b) température dans la plage de 37 à 42 degrés C, et (c) oxygène dissous : 20 à 80 % de saturation, de préférence 20 à 40 % de saturation ; et ladite culture est conduite pendant une durée de 4 à 10 heures, de préférence 6 heures.

7. Procédé selon l'une quelconque des revendications précédentes, dans lequel l'expression de protéine OSMnat recombinante est induite après avoir atteint une accumulation de biomasse appropriée en utilisant l'inducteur d'opéron lac adapté IPTG à une concentration de 0,1 mM à 1 mM, de préférence 0,25 mM.

8. Procédé selon l'une quelconque des revendications précédentes, dans lequel l'OSM dans les étapes a) à c) est l'oncostatine M méthionylée recombinante humaine (rmetHuOSM).

9. Procédé selon l'une quelconque des revendications précédentes, dans lequel, dans l'étape b), l'OSM dans le matériau insoluble est solubilisée en utilisant un agent chaotrope, de préférence à une concentration intermédiaire d'un agent chaotrope, à un pH dans la plage 8,00 à 9,00.

10. Procédé selon la revendication 9, comprenant en outre la séparation de l'OSM repliée à partir du chaotrope, dans lequel l'OSM repliée est séparée du chaotrope par filtration sur gel, dans lequel la colonne de filtration sur gel est, de préférence, Sephadex G-25.

11. Procédé selon l'une quelconque des revendications précédentes, dans lequel la solubilisation dans l'étape b) est effectuée au moyen de chlorhydrate de guanidinium, la concentration de chlorhydrate de guanidinium étant de 3,0 à 7,0, de préférence 6,0 M.

12. Procédé selon l'une quelconque des revendications précédentes, dans lequel la chromatographie dans l'étape d) est une chromatographie d'échange d'ions en une étape effectuée sur une colonne de CM-Sepharose ou SP-Sepharose à un pH de 5,2 à 5,6.

13. Procédé selon l'une quelconque des revendications précédentes, dans lequel la protéine OSM purifiée obtenue est OSM totalement native de 196 acides aminés.
